# EUROPEAN PATENT APPLICATION

(11) **EP 4 378 377 A1**
(43) Date of publication of application: **05.06.2024**
(21) Application number: 22864825.9
(22) Date of filing: 02.06.2022
(51) Int. Cl.: A61B 5/00, A61B 5/145, A61B 5/155

(54) **PROTECTION SHEET FOR BODY ATTACHMENT DEVICE AND BODY ATTACHMENT DEVICE INCLUDING SAME**

(30) Priority: 01.09.2021 KR 20210116129
(71) Applicant: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2022/007874
(87) International publication number: WO 2023/033316

(57) **Abstract**

A protection sheet for a body attachment device according to the present invention relates to a protection sheet for a body attachment device, which is detachably coupled to an adhesive layer to cover and protect the adhesive layer of the body attachment device, which is attached to the skin of a user, the protection sheet comprising: a first section and a second section partitioned by a cutting line which is disposed inside an outer rim of the protection sheet; and an adhesive part provided on at least one of the first section and the second section so that a separation member by which the protection sheet can be pulled in a direction in which the protection sheet can be separated from the adhesive layer can be attached.

## Description

### TECHNICAL FIELD

The present disclosure relates to a body attachable device, and more specifically, to a protective sheet for a body attachable device which covers the adhesive layer provided on the body attachable device to be attached to the skin of a user, and a body attachable device comprising the protective sheet.

### BACKGROUND

With the recent advancement of medical technology, various body attachable devices that are attached to the body of a user have been developed and sold. The body attachable devices can be useful for monitoring biometric information or providing treatment by attaching them to the body of a patient with a chronic disease.

For example, chronic diseases such as diabetes require continuous management, and the body attachable unit can be used to manage blood glucose levels in diabetic patients.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable device that has a sensor which is inserted into the skin of the user to measure blood glucose levels, a terminal that outputs the received blood glucose levels, and so on. The sensor is coupled with a transmitter to form a body attachable device that wirelessly transmits biometric information to a terminal, etc.

The body attachable device of the continuous glucose measurement system has an adhesive layer and can measure the blood glucose level for several days to tens of days by being attached to the skin of a user by an applicator. The adhesive layer of the body attachable device is covered and protected with a protective sheet, and the protective sheet is removed before the body attachable device is attached to the skin of a user.

Generally, a body attachable device of a continuous blood glucose measurement system is provided to a user in an assembled state with an applicator. Therefore, in order to operate the applicator and attach the body attachable device to the skin of a user, the protective sheet covering the adhesive layer of the body attachable device must first be removed.

### SUMMARY

### Technical Problem

The present disclosure is developed in consideration of the above-mentioned points, and the purpose is to provide a protective sheet for a body attachable device that covers and protects the adhesive layer of the body attachable device, and can be smoothly separated from the adhesive layer by a user.

Another purpose of the present disclosure is to provide a body attachable device that is convenient to use in that the protective sheet that covers and protects the adhesive layer can be easily and stably separated from the adhesive layer by a user.

### Solution to Problem

To accomplish the above-described purposes, according to an embodiment of the present disclosure, a protective sheet for a body attachable device detachably coupled to an adhesive layer to cover and protect the adhesive layer of the body attachable device configured to be attachable to skin of a user may comprise: a first section and a second section divided by a cut line disposed inside an outer edge portion of the protective sheet; and an adhesive portion provided at at least one of the first section and the second section such that a separation member configured to be pullable in a direction of separating the protective sheet from the adhesive layer is adhered.

The protective sheet for the body attachable device according to an embodiment of the present disclosure may comprise a connection section connecting the first section and the second section, wherein the first section and the second section may be configured to be separatable from the adhesive layer in a state of being connected to each other by the connection section while being sequentially separatable from the adhesive layer by the separation member.

The connection section may comprise a middle section disposed inside the second section and surrounding the first section; a first section connection portion connecting the first section and the middle section; and a middle connection portion connecting the middle section and the second section, and the cut line may comprise a first section cut line dividing the first section and the middle section; and a middle section cut line dividing the second section and the middle section.

The first section cut line may comprise a first section end cut portion dividing one end of the first section and the middle section; and a pair of first section side cut portions extending from both ends of the first section end cut portion toward the outer edge portion, respectively, and the middle section cut line may comprise a middle end cut portion dividing one end of the middle section and the second section; and a pair of middle side cut portions extending from both end portions of the middle end cut portion toward the outer edge portion, respectively.

The first section cut line may comprise a pair of first section connection end cut portions connected to the pair of first section side cut portions, respectively, to be disposed to be symmetrical to each other with respect to the first section connection portion, the middle section cut line may comprise a pair of middle section connection end cut portions connected to the pair of middle side cut portions, respectively, to be disposed to be symmetrical to each other with respect to the middle connection portion, and the pair of first section connection end cut portions and the pair of middle section connection end cut portions may be formed in a curved shape.

The second section may be arranged to surround the first section, and the cut line may comprise a first section end cut portion dividing one end of the first section and the second section; and a pair of first section side cut portions extending from both ends of the first section end cut portion toward the outer edge portion, respectively.

The cut line may comprise a cross cut portion extending from the outer edge portion toward the first section and connected to one of the pair of first section side cut portions.

The second section may be arranged to surround the first section, and the cut line may comprise a first cut portion having a curved shape surrounding the first section, and a second cut portion connected to the first cut portion and extending to the outer edge.

The first section and the second section may be arranged such that respective ends of the first section and the second section face each other, and the connection section is arranged to face a side of the first section and a side of the second section, and the cut line may comprise a main cut portion dividing the connection section into the side portion of the first section and the side portion of the second section; and a cross cut portion connected to the main cut portion by extending from the outer edge portion toward the main cut portion to divide the end of the first section and the end of the second section.

A side of the first section and a side the second section may be arranged to face each other, and the cut line may extend from one side of the outer edge portion to the connection section to divide the side of the first section and the side of the second section.

The cut line may extend from one side of the outer edge portion to an other side of the outer edge portion such that the first section and the second section are individually separatable from the adhesive layer without any part connected to each other, and the adhesive portion may be provided in the first section and the second section, respectively.

The first section and the second section may be arranged to be point-symmetrical with respect to a center of the protective sheet.

The protective sheet for the body attachable device according to an embodiment of the present disclosure may comprise: a protective sheet hole formed inside the outer edge portion to penetrate the protective sheet in a thickness direction so that a sensor, included in the body attachable device to be insertable into the skin of the user, passes therethrough.

To accomplish the above-described purposes, according to an embodiment of the present disclosure, a body attachable device configured to be attachable to skin of a user may comprise: a housing; an adhesive layer provided at one side of the housing to be attachable to the skin of the user; and a protective sheet detachably coupled to the adhesive layer to cover and protect the adhesive layer, wherein the protective sheet comprises a first section and a second section divided by a cut line disposed inside an outer edge portion of the protective sheet; and an adhesive portion provided at at least one of the first section and the second section such that a separation member configured to be pullable in a direction of separating the protective sheet from the adhesive layer is adhered.

### Advantageous Effects of Invention

According to the present disclosure, since the protective sheet that covers and protects the adhesive layer of the body attachable device is divided into separately removable first and second sections, the protective sheet can be easily detached from the adhesive layer.

Additionally, according to the present disclosure, when the protective sheet is pulled in a direction in which the protective sheet is separated from the adhesive layer, the protective sheet can be stably separated from the adhesive layer without tearing.

### BRIEF DESCRIPTION OF THE DRAWINGS

FIG. 1 is a side view showing a body attachable device according to an embodiment of the present disclosure.
FIG. 2 is a bottom view showing a protective sheet of a body attachable device shown in FIG. 1.
FIG. 3 shows a body attachable device shown in FIG. 1 mounted inside an applicator.
FIGS. 4 to 6 show a process of separating a protective sheet from an adhesive layer of a body attachable device mounted on an applicator.
FIGS. 7 to 10 show a process of attaching a body attachable device to the skin of a user using an applicator.
FIGS. 11 to 20 show various modifications of a protective sheet for a body attachable device.

### DETAILED DESCRIPTION OF EMBODIMENTS

Hereinafter, a protective sheet for a body attachable device according to the present disclosure and a body attachable device comprising the protective sheet will be described in detail with reference to the drawings.

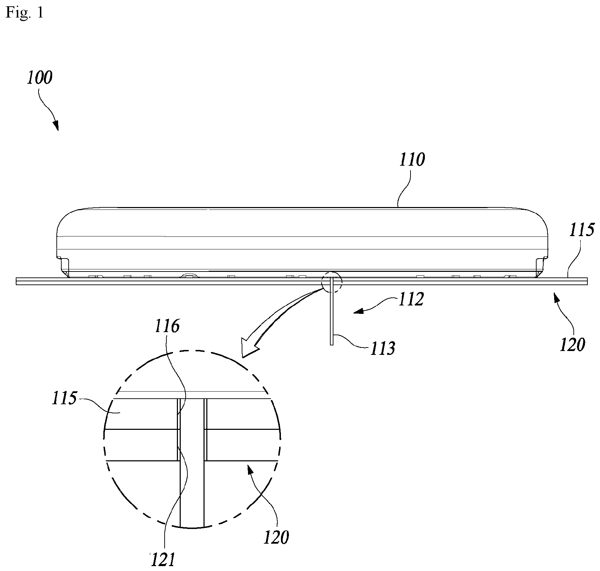

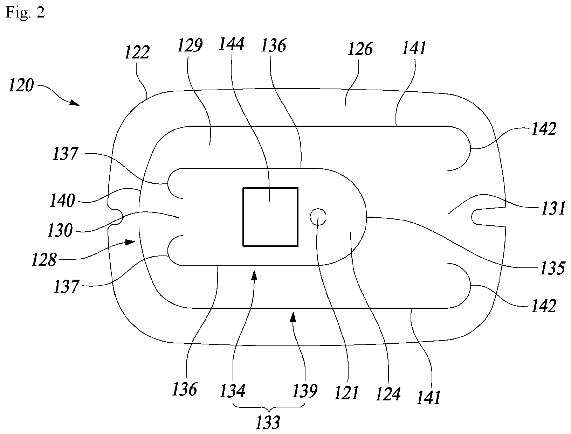

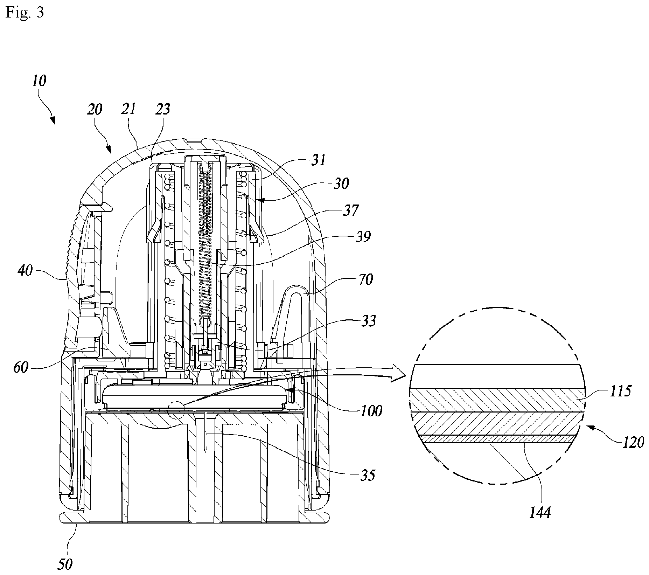

FIG. 1 is a side view showing a body attachable device according to an embodiment of the present disclosure, FIG. 2 is a bottom view showing a protective sheet of the body attachable device shown in FIG. 1, and FIG. 3 shows a body attachable device shown in FIG. 1 mounted on the inside of an applicator.

The body attachable device (100) according to an embodiment of the present disclosure can be attached to the skin of a user and measure the biometric information of a user. The body attachable device (100) may include a measuring unit for measuring the user's biometric information, and various electronic components for processing the biometric information measured by the measuring unit or transmitting the measured biometric information. Biometric information that the body attachable device (100) can measure is not limited to specific information and can be diverse. In this embodiment, the body attachable device (100) is described, for example, as being attached to the skin of a user to measure the blood glucose level of a user, and transmits the blood glucose measurement information to an external terminal.

The body attachable device (100) includes a housing (110), a sensor (112) as a measuring unit for measuring the blood glucose of a user, an adhesive layer (115) which is provided on one side of the housing (110) to be attached to the skin of a user (S), and a protective sheet (120) which covers and protects the adhesive layer (115). Various electronic components may be installed inside the housing (110) to process blood glucose information measured by the sensor (112) and transmit it to the outside.

The sensor (112) is partially accommodated inside the housing (110) and is electrically connected to electronic components disposed inside the housing (110). Additionally, the sensor (112) includes an insertion portion (113) that protrudes from the housing (110) to be inserted into the skin of a user.

The adhesive layer (115) is provided on the bottom surface of the housing (110) to be adhered to the skin of a user (S). In the middle portion of the adhesive layer (115), an adhesive layer hole (116) through which the insertion portion (113) of the sensor (112) can pass is formed to penetrate the adhesive layer (115) in the thickness direction.

In the drawing, the adhesive layer (115) is shown to have a larger area than the bottom surface of the housing (110), but the size or shape of the adhesive layer (115) can be changed in various ways.

The protective sheet (120) covers and protects the adhesive surface of the adhesive layer (115). In the middle part of the protective sheet (120), a protective sheet hole (121) corresponding to the adhesive layer hole (116) of the adhesive layer (115) is formed to penetrate the protective sheet (120) in the thickness direction. The insertion portion (113) of the sensor (112) may pass through the protective sheet hole (121) and protrude to the outside of the protective sheet (120). The protective sheet (120) includes a first section (124) and a second section (126) that can be individually separated from the adhesive layer (115) . The first section (124) and the second section (126) are divided by a cut line (133) disposed inside the outer edge portion (122) of the protective sheet (120). The first section (124) and the second section (126) are connected by a connection section (128). The connection section (128) is disposed inside the second section (126) to surround the first section (124). The protective sheet (120) is configured such that the second section (126) is disposed on the outermost side to surround the connection section (128), the connection section (128) is disposed between the first section (124) and the second section (126) to surround the first section (124), and the first section (124) is disposed on the innermost side. The first section (124) is provided with a protective sheet hole (121) and an adhesive portion (144). The adhesive portion (144) may be in the form of a double-sided tape.

A separation member that can be pulled in a direction to separate the protective sheet (120) from the adhesive layer (115) may be attached to the adhesive portion (144). The first section (124) and the second section (126) can be sequentially separated from the adhesive layer (115) by a separation member, and can be separated from the adhesive layer (115) in a state of being connected to each other by the connection section (128). When the separation member is pulled while attached to the adhesive portion 144, the first section (124) is first separated from the adhesive layer (115), and then the connection section (128) and the second section (126) may be sequentially separated from the adhesive layer (115).

The connection section (128) includes a middle section (129) which is disposed inside the second section (126) and surrounds the first section (124), a first section connection portion (130) connecting the first section (124) and the middle section (129), and a middle connection portion (131) connecting the middle section (129) and the second section (126).

The cut line (133) includes a first section cut line (134) dividing the first section (124) and the middle section (129), and a middle section cut line (139) separating the second section (126) and the middle section (129).

The first section cut line (134) includes a first section end cut portion (135) that divides one end of the first section (124) and the middle section (129), a pair of first section side cut portions (136) each connected to respective ends of the first section end cut portion (135), and a pair of first section connection end cut portions (137) provided at each end of the pair of first section side cut portions (136). The first section end cut portion (135) may be arched, curved in another shape, or straight. In a case in which the first section end cut portion (135) is curved as shown, when the first section (124) is pulled in a direction in which the first section (124) is separated from the adhesive layer (115) by the separation member, the first section (124) can be more smoothly separated from the connection section (128) without being torn. The pair of first section side cut portions (136) may respectively extend in a straight or curved shape from both ends of the first section end cut portion (135) toward the outer edge portion (122). In the drawing, a pair of first section side cut portions (136) are shown in a straight line and arranged parallel to each other, but the pair of first section side cut portions (136) may be curved or arranged in a direction of mutual convergence. A pair of first section connection end cut portions (137) are arranged to be symmetrical to each other with respect to the first section connection portion (130). The first section connection end cut portion (137) may be arched, curved in another shape, or straight. In a case in which the first section connection end cut portion (137) is curved as shown, when the first section (124) is pulled in a direction in which the first section (124) is separated from the adhesive layer (115) by the separation member, the first section (124) can be more smoothly separated from the connection section (128) without being torn.

The middle section cut line (139) includes a middle end cut portion (140) that divides one end of the middle section (129) and the second section (126), a pair of middle section side cut portions (141) each connected to respective ends of the middle section side cut portion (140), and a pair of middle section connection end cut portions (142) provided at each end of the pair of middle section side cut portions (141). The middle end cut portion (140) may be arched, curved in another shape, or straight. In a case in which the middle end cut portion (140) is curved as shown, when the connection section (128) is pulled in a direction in which the connection section (128) is separated from the adhesive layer (115) by the first section (124), the connection section (128) can be more smoothly separated from the second section (126) without being torn. The pair of middle section side cut portions (141) may respectively extend in a straight or curved shape from both ends of the middle end cut portion (140) toward the outer edge portion (122). In the drawing, a pair of middle section side cut portions (141) are shown in a straight line and arranged parallel to each other, but the pair of middle section side cut portions (141) may be curved or arranged in a direction of mutual convergence. The pair of middle section connection end cut portions (142) are arranged to be symmetrical to each other with respect to the middle connection portion (131). The middle section connection end cut portion (142) may be arched or curved in another shape, or straight. In a case in which the middle section connection end cut portion (142) is curved as shown, when the connection section (128) is pulled in a direction in which the connection section (128) is separated from the adhesive layer (115) by the first section (124), the connection section (128) can be more smoothly separated from the second section (126) without being torn.

The pair of middle section connection end cut portions (142) are disposed closer to the first section end cut portion (135) than the middle end cut portion (140). Accordingly, when the protective sheet (120) is pulled by the separation member, one end of the first section (124) begins to separate from the adhesive layer (115) first, and then after the entire first section (124) is separated, one end of the first section connection portion (130) and the middle section (129) begins to be separated, and after the entire middle section (129) is separated, the middle connection portion (131) and one end of the second section (126) begin to separate, so that the entire second section (126) can be separated. Therefore, it is possible to smoothly separate the entire protective sheet (120) from the adhesive layer (115) with relatively small force.

The protective sheet (120) of the body attachable device (100) according to this embodiment is not limited to the configuration shown and may be changed in various ways. For example, the shape of each of the first section (124), the second section (126), and the connection section (128) may be changed in various ways. And the positions of the first section connection portion (130) and the middle connection portion (131) may be changed. Additionally, in the drawing, one adhesive portion (144) is shown as being provided in the first section (124), but the number or location of the adhesive portions (144) may be changed in various ways. Additionally, the formation location of the protective sheet hole (121) may be changed.

Hereinafter, with reference to FIGS. 3 to 10, a process of attaching the body attachable device (100) according to an embodiment of the present disclosure to the skin of a user (S) using the applicator (10) will be described.

As shown in FIG. 3, the body attachable device (100) can be mounted on the applicator (10) and attached to the skin of a user (S) by the applicator (10). The applicator (10) includes an applicator housing (20) and an insertion unit (30) for moving the body attachable device (100) from a first position to a second position, an operating member (40) for user manipulation, and a protective cap (50) detachably coupled to the open end of the applicator housing (20). Here, the first position is a position where the body attachable device (100) is spaced a certain distance from the open end of the applicator housing (20), and the second position is a position where the body attachable device (100) moves a certain distance from the first position so that the insertion portion (113) of the sensor (112) can be inserted into the skin of a user (S). The applicator housing (20) includes an outer case (21) having one end in contact with the skin of a user (S), and an inner case (23) disposed inside the outer case (21) to support the insertion unit (30). The insertion unit (30) is installed in the inner case (23). The insertion unit (30) includes a shuttle (31) that supports the body attachable device (100) and can move from a first position to a second position, a carrier (33) that is relatively movably coupled to the shuttle (31) and supports the needle (35), a spring (37) for providing a moving force to the shuttle (31) and a spring (39) for providing a moving force to the carrier (33). The needle (35) is arranged to penetrate the housing (110) of the body attachable device (100) so as to be inserted into the skin of a user (S) together with the insertion portion (113) of the sensor (112). The needle (35) may pass through the adhesive layer hole (116) of the adhesive layer (115) and the protective sheet hole (121) of the protective sheet (120), and then protrude from the housing (110). The needle (35) is coupled to the carrier (33) and moves from the first position to the second position with the shuttle (31) to be inserted into the skin of a user (S). The shuttle (31) can be moved from the first position to the second position by the spring (37). The carrier (33) can move in a direction to separate the needle (35) from the skin of a user (S) by the spring (39) after the needle (35) and the insertion portion (113) are inserted into the skin of a user (S). The protective cap (50) is detachably coupled to the open end of the applicator housing (20) to protect the insertion portion (113) of the sensor (112) and the needle (35) from being exposed to the outside. Additionally, the protective cap (50) may function as a separation member to separate the protective sheet (120) from the adhesive layer (115). In a state in which the protective cap (50) is coupled to one end of the applicator housing (20), one side of the protective cap (50) is attached to the adhesive portion (144) of the protective sheet (120) and connected to the protective sheet (120). The protective cap (50) can be pulled in a direction to separate the protective sheet (120) from the adhesive layer (115) when separated from the applicator housing (20) by a user.

In addition, the applicator (10) includes a stopper member (60) for maintaining the shuttle (31) in the first position, and a return member (70) for elastically supporting the stopper member (60). The stopper member (60) may maintain the shuttle (31) in the first position by engaging the shuttle (31) located in the first position, and may be moved in a direction to disengage with the shuttle (31) by the operating member (40).

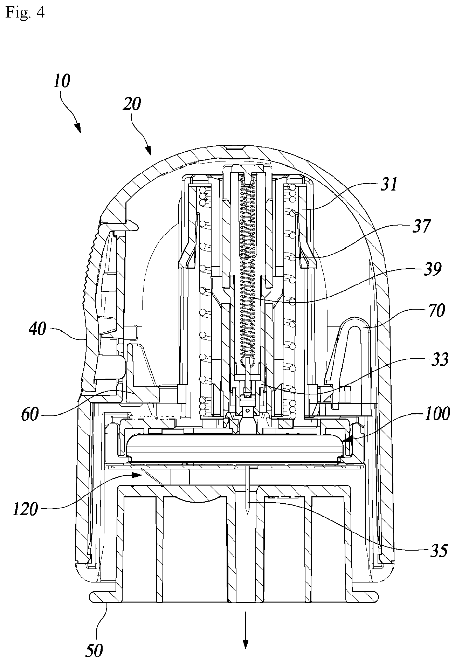

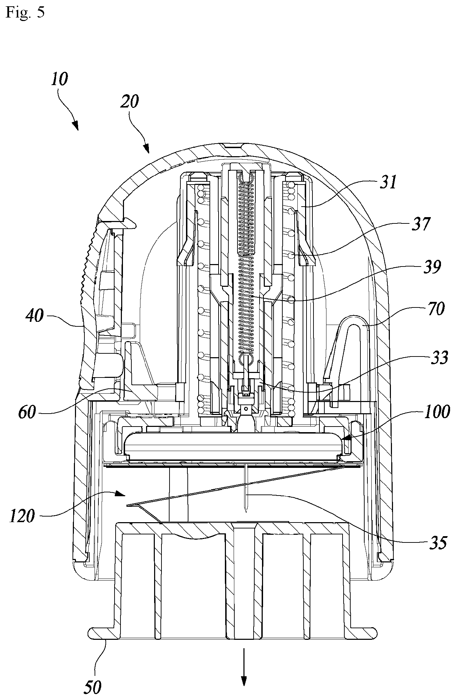

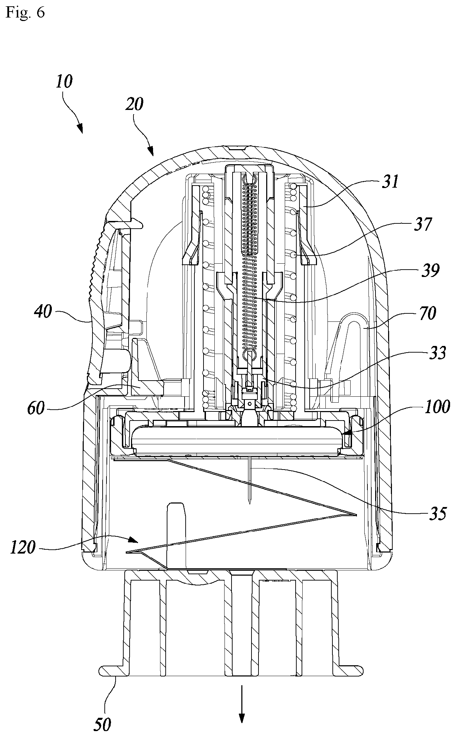

In order to attach the body attachable device (100) to the skin of a user (S), the protective sheet (120) must first be separated from the adhesive layer (115). The protective sheet (120) may be separated by the protective cap (50) in a state in which the body attachable device (100) is attached to the applicator (10). The process of separating the protective sheet (120) using the protective cap (50) is the same as shown in FIGS. 4 to 6. As shown in FIG. 4, when a user pulls the protective cap (50) in a direction to separate it from the applicator housing (20), the first section (124) of the protective sheet (120) is first separated from the adhesive layer (115). As a user pulls the protective cap (50) further to separate it from the applicator housing (20), the middle section (129) of the protective sheet (120) separates from the adhesive layer (115), as shown in FIGS. 5 and 6, and finally, the second section (126) of the protective sheet (120) may be separated from the adhesive layer (115). In this way, because the first section (124), the middle section (129) and the second section (126) of the protective sheet (120) can be separated from the adhesive layer (115) in turn, it is possible for a user to separate the protective sheet (120) from the adhesive layer (115) with relatively small force.

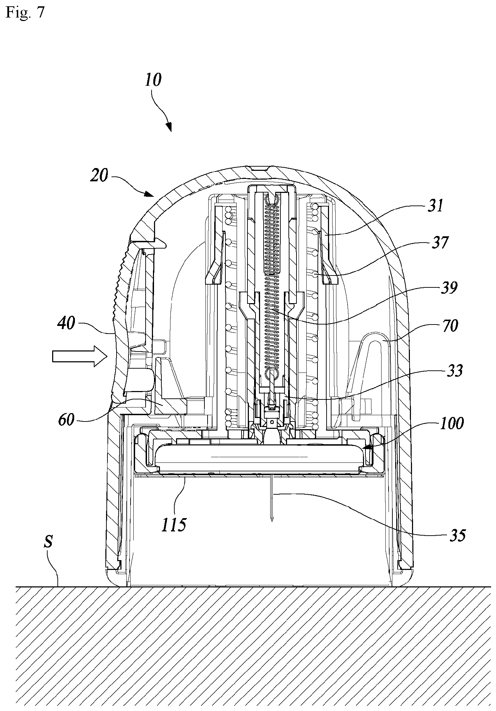

After the protective sheet (120) is removed and the adhesive layer (115) of the body attachable device (100) is exposed, the body attachable device (100) can be attached to the skin of a user (S) using the applicator (10). A specific method of attaching the body attachable device (100) to the skin of a user (S) using the applicator (10) is as shown in FIGS. 7 to 10.

First, as shown in FIG. 7, one end of the applicator housing (20) is placed on the skin of a user (S) and the operating member (40) is manipulated. When the operating member (40) is pressed and operated, the stopper member (60) moves in a direction to disengage with the shuttle (31).

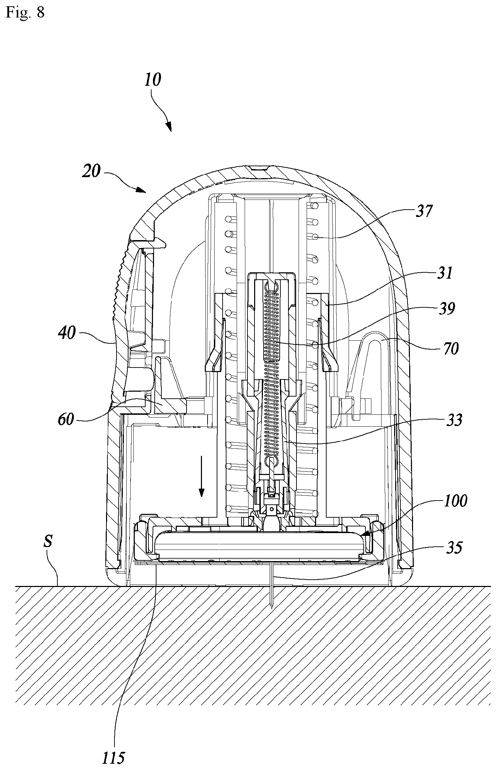

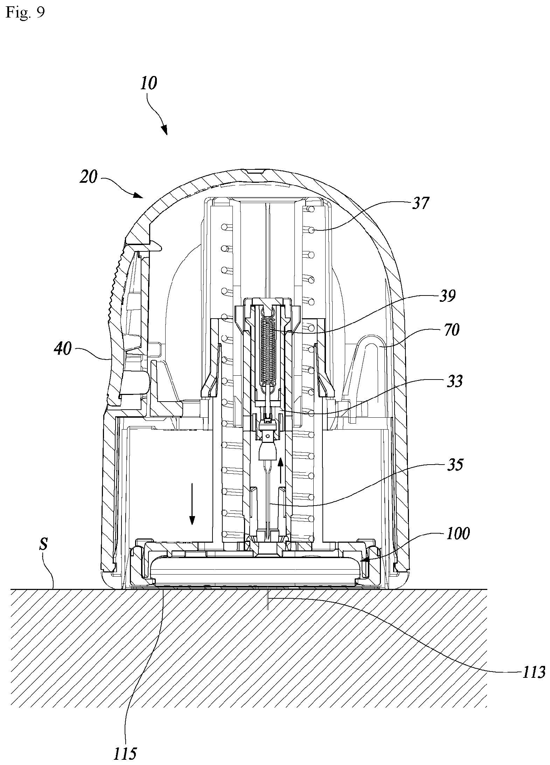

When the stopper member (60) is disengaged from the shuttle (31), the shuttle 31 is moved from the first position toward the second position by the spring (37), as shown in FIG. 8. At this time, the needle (35) and the insertion portion (113) of the sensor (112) are inserted into the skin of a user (S). As shown in FIG. 9, when the body attachable device (100) reaches the second position, the body attachable device (100) is attached to the skin of a user (S) by the adhesive layer in a state in which the insertion portion (113) of the sensor (112) is inserted into the skin of a user (S). And in a state in which the insertion portion (113) of the sensor (112) is inserted into the skin of a user (S), the carrier (33) is disengaged from the shuttle (31) and moves toward the first position by the spring (39). Accordingly, the needle (35) is separated from the skin of a user (S).

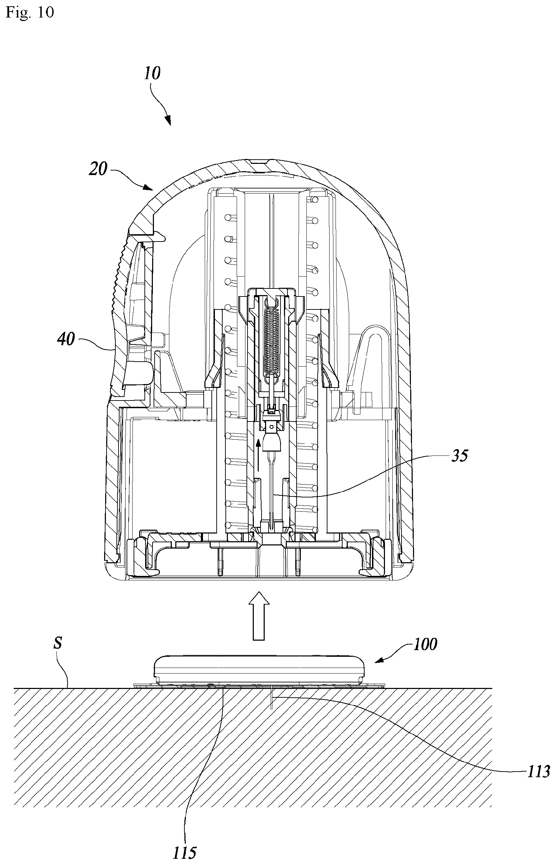

After the needle (35) is separated from the skin of a user (S), a user may separate the applicator (10) from the body attachable device (100) as shown in FIG. 10. The body attachable device (100) attached to the skin of a user (S) can measure the blood glucose level of a user and transmit the measurement information to an external terminal, etc.

The applicator (10) used to attach the body attachable device (100) according to an embodiment of the present disclosure to the skin of a user (S) is not limited to the form shown and may be changed in various ways. Additionally, the drawing shows that the protective cap (50), which is detachably coupled to the open end of the applicator housing (20), is used as a separation member to separate the protective sheet (120), but various separation members other than the protective cap (50) may be used to separate the protective sheet (120).

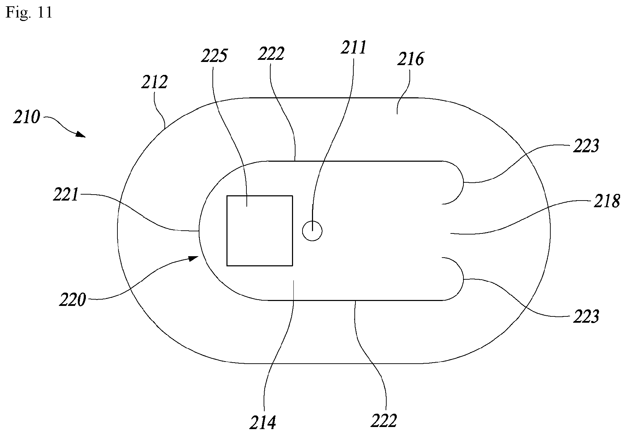

Meanwhile, FIGS. 11 to 20 show various modified examples of protective sheets for body attachable devices.

First, the protective sheet (210) shown in FIG. 11 includes a protective sheet hole (211) through which the insertion portion (113) of the sensor (112) can pass, and a first section (214) and a second section (216) defined by a cut line (220) which is disposed inside the outer edge portion (212). The first section (214) and the second section (216) are connected by a connection section (218). The protective sheet (210) is formed such that the second section (216) surrounds the connection section (218). The first section (214) includes a protective sheet hole (211) and an adhesive portion (225). A separation member for separating the protective sheet (210) from the adhesive layer (115) may be attached to the adhesive portion (225). The first section (214) and the second section (216) can be sequentially separated from the adhesive layer (115) by a separation member, and can be separated from the adhesive layer (115) in a state of being connected to each other by the connection section (218). When the separation member is pulled while attached to the adhesive portion (225), the first section (214) may be first separated from the adhesive layer (115), and then the second section (216) may be separated from the adhesive layer (115).

The cut line (220) includes a first section end cut portion (221) that divides one end of the first section (214) and the second section (216), a pair of first section side cut portions (222) each connected to both ends of the first section end cut portion (221), and a pair of first section connection end cut portions (223) provided at each end of the pair of first section side cut portions (222). The first section end cut portion (221) may be arched, curved in another shape, or straight. In a case in which the first section end cut portion (221) is curved as shown, when the first section (214) is pulled in a direction in which the first section (214) is separated from the adhesive layer (115) by the separation member, the first section (214) may be more smoothly separated from the second section (216) without being torn. The pair of first section side cut portions (222) may respectively extend in a straight or curved shape from both ends of the first section end cut portion (221) toward the outer edge portion (212). In the drawing, the pair of first section side cut portions (222) are shown to be straight and arranged parallel to each other, but the pair of first section side cut portions (222) may be curved or arranged in a direction of mutual convergence. The pair of first section connection end cut portions (223) are arranged to be symmetrical to each other with respect to the connection section (218). The first section connection end cut portion (223) may be arched, curved in another shape, or straight. In a case in which the first section connection end cut portion (223) is curved as shown, when the first section (214) is pulled in a direction in which the first section (214) is separated from the adhesive layer (115) by the separation member, the first section (214) may be more smoothly separated from the second section (216) without being torn.

The shapes of each of the first section (214), second section (216), and connection section (218) of the protective sheet (210) according to this embodiment are not limited to those shown and may be changed in various ways. Additionally, the number or location of the adhesive portions (225) may be changed in various ways. Additionally, the formation location of the protective sheet hole (211) may be changed.

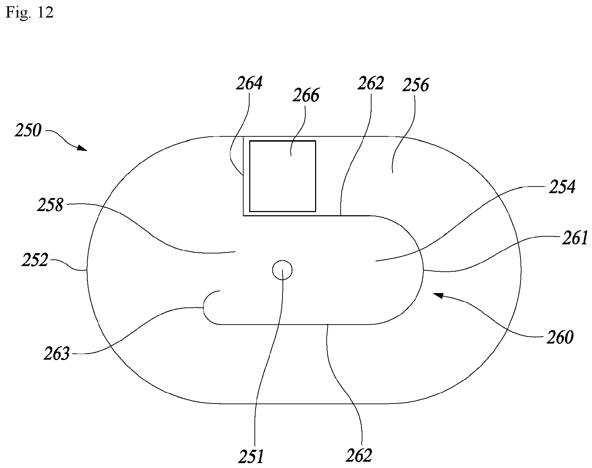

The protective sheet (250) shown in FIG. 12 includes a protective sheet hole (251) through which the insertion portion (113) of the sensor (112) can pass, a first section (254) and a second section (256) delimited by a cut line (260) disposed inside the outer edge portion (252), and an adhesive portion (266). The first section (254) and the second section (256) are connected by a connection section (258). The protective sheet (250) is formed such that a second section (256) surrounds the connection section (258). The first section (254) includes a protective sheet hole (251), and the second section (256) includes an adhesive portion (266). A separation member for separating the protective sheet (250) from the adhesive layer (115) may be attached to the adhesive portion (266).

The first section (254) and the second section (256) can be sequentially separated from the adhesive layer (115) by a separation member, and can be separated from the adhesive layer (115) in a state of being connected to each other by the connection section (258).

The cut line (260) includes a first section end cut portion (261) that divides one end of the first section (254) and the second section (256), a pair of first section side cut portions (262) each connected to both ends of the first section end cut portion (261), a first section connection end cut portion (263) provided at one end of the pair of first section side cut portions (262), and a cross cut portion (264) connected to the other of the pair of first section side cut portions (262). The cross cut portion (264) extends from the outer edge portion (252) toward the first section (254) and is connected to the end of one first section side cut portion (262). The first section end cut portion (261) may be arched or curved in another shape, or straight. In a case in which the first section end cut portion (261) is curved as shown, when the first section (254) is pulled in a direction in which the first section (254) is separated from the adhesive layer (115) by the separation member, the first section (254) may be more smoothly separated from the second section (256) without being torn. The pair of first section side cut portions (262) may extend in a straight or curved shape from both ends of the first section end cut portion (261) toward the outer edge portion (252), respectively. In the drawing, the pair of first section side cut portions (262) are shown in a straight line and arranged parallel to each other, but the pair of first section side cut portions (262) may be curved or arranged in a direction of mutual convergence. The first section connection end cut portion (263) may be arched, curved in another shape, or straight. In a case in which the first section connection end cut portion (263) is curved as shown, when the first section (254) is pulled in a direction in which the first section (254) is separated from the adhesive layer (115) by the separation member, the first section (254) may be more smoothly separated from the second section (256) without being torn.

The adhesive portion (266) is provided at the end of the second section (256) so as to be adjacent to the cross cut portion (264). When the separation member is pulled in a state of being attached to the adhesive portion (266), the end of the second section (256) first begins to separate from the adhesive layer (115), and after the entire second section (256) is separated from the adhesive layer (115), the first section (254) can be separated from the adhesive layer (115).

The shapes of each of the first section (254), second section (256), and connection section (258) of the protective sheet (250) according to this embodiment are not limited to those shown and may be changed in various ways. Additionally, the number or location of the adhesive portions (266) may be changed in various ways. Additionally, the formation location of the protective sheet hole (251) may be changed. Additionally, the cross cut portion (264) may be curved or may be diagonally connected to the first section side cut portion (262).

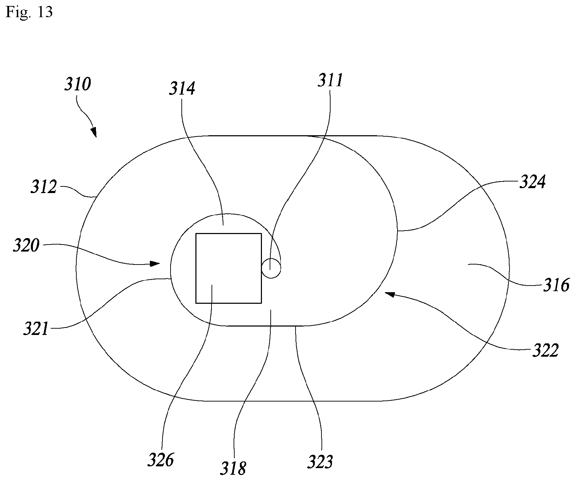

The protective sheet (310) shown in FIG. 13 includes a protective sheet hole (311) through which the insertion portion (113) of the sensor (112) can pass, and a first section (314) and a second section (316) divided by a cut line (320) disposed inside the outer edge portion (312). The first section (314) and the second section (316) are connected by a connection section (318). The protective sheet (310) is formed such that the second section (316) surrounds the first section (314). The first section (314) includes a protective sheet hole (311) and an adhesive portion (326). A separation member for separating the protective sheet (310) from the adhesive layer (115) may be attached to the adhesive portion (326). The first section (314) and the second section (316) can be sequentially separated from the adhesive layer (115) by a separation member, and can be separated from the adhesive layer (115) in a state of being connected to each other by the connection section (318). When the separation member is pulled in a state of being attached to the adhesive portion (326), the first section (314) is first separated from the adhesive layer (115), and then the second section (316) can be separated from the adhesive layer (115).

The cut line (320) includes a curved first cut portion (321) surrounding the first section (314), and a second cut portion (322) that is connected to the first cut portion (321) and extends to the outer edge portion (312). In the drawing, the second cut part (322) is shown as including a straight portion (323) that is connected to the first cut portion (321) and a curved portion (324) that is connected to the straight portion (323) and extends to one side of the outer edge portion (312), but the shape of the second cut portion (322) can be changed in various ways.

When the separation member is pulled in a state of being attached to the adhesive portion (326), the first section (314) is first separated from the adhesive layer (115), and then the spiral-shaped second section (316) can be sequentially separated from the inner portion.

The shapes of each of the first section (314), second section (316), and connection section (318) of the protective sheet (310) according to this embodiment are not limited to those shown and may be changed in various ways. Additionally, the number or location of the adhesive portions (326) may be changed in various ways. Additionally, the formation location of the protective sheet hole (311) may be changed.

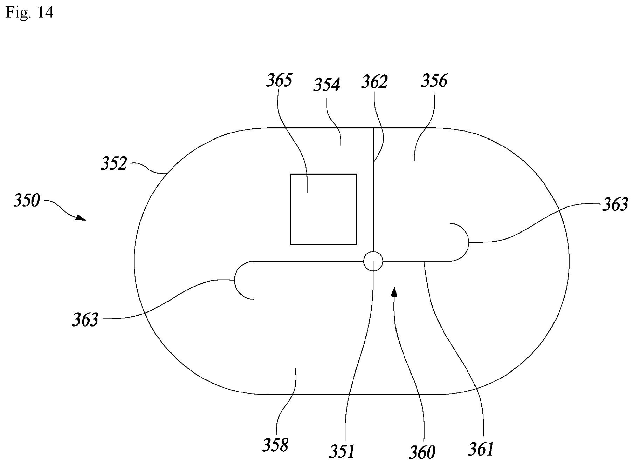

The protective sheet (350) shown in FIG. 14 includes a protective sheet hole (351) through which the insertion portion (113) of the sensor (112) can pass, and a first section (354) and a second section (356) divided by a cut line (360) disposed inside the outer edge portion (352). The first section (354) and the second section (356) are connected by a connection section (358). The first section (354) and the second section (356) are arranged so that their respective ends face each other, and the connection section (358) is disposed to face the side of the first section (354) and the side of the second section (356), respectively. The protective sheet hole (351) is formed in a portion where the first section (354), the second section (356), and the connection section (358) overlap. The first section (354) includes an adhesive portion (365). A separation member for separating the protective sheet (350) from the adhesive layer (115) may be attached to the adhesive portion (365). The first section (354) and the second section (356) can be sequentially separated from the adhesive layer (115) by a separation member, and can be separated from the adhesive layer (115) in a state of being connected to each other by the connecting section (358). When the separation member is pulled in a state of being attached to the adhesive portion (365), the first section (354) is first separated from the adhesive layer (115), and then the connection section (358) and the second section (356) can be sequentially separated from the adhesive layer (115).

The cut line (360) includes a main cut portion (361) that separates the connection section (358) from the side of the first section (354) and the side of the second section (356), a cross cut portion (362) that divides the ends of the first section (354) and the ends of the second section (356), and a pair of sub-cut portions (363) respectively connected to both ends of the main cut portion (361). One of the sub-cut portions (363) is located where the first section (354) and the connection section (358) are connected, and the other is located where the connection section (358) and the second section (356) are connected. The main cut portion (361) is formed in a straight line passing through the protective sheet hole (351). The cross cut portion (362) extends from the outer edge portion (352) toward the main cut portion (361) and is connected to the main cut portion (361). The cross cut portion (362) may be connected to the main cut portion (361) through the protective sheet hole (351) by being connected to the protective sheet hole (351). The pair of sub-cut portions (363) may be arched, curved in another shape, or straight. In a case in which the sub-cut portion (363) is curved as shown, when the first section (354) is pulled in a direction in which the first section (354) is separated from the adhesive layer (115) by the separation member, the first section (354) can be more smoothly separated from the connection section (358) without being torn, and the connection section (358) can be more smoothly separated from the second section (356) without being torn.

The shapes of each of the first section (354), second section (356), and connection section (358) of the protective sheet (350) according to this embodiment are not limited to those shown and may be changed in various ways. Additionally, the number or location of the adhesive portions (365) may be changed in various ways. Additionally, the formation location of the protective sheet hole (351) may be changed. Additionally, the drawing shows that the main cut portion (361) and the cross cut portion (362) are arranged perpendicular to each other, but the arrangement angles of each of the main cut portion (361) and the cross cut portion (362) may be changed in various ways. Additionally, the main cut portion (361) or the cross cut portion (362) may be curved.

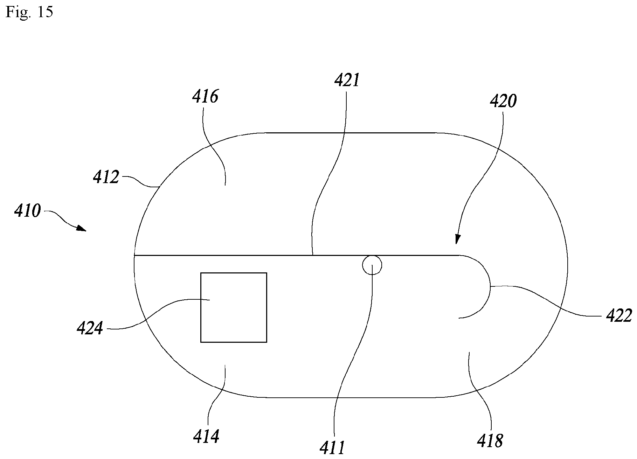

The protective sheet (410) shown in FIG. 15 includes a protective sheet hole (441) through which the insertion portion (113) of the sensor (112) can pass, and a first section (414) and a second section (416) divided by a cut line (420) disposed inside the outer edge portion (412). The first section (414) and the second section (416) are connected by a connection section (418). The first section (414) and the second section (416) are arranged so that their respective sides face each other. The first section (414) is provided with a protective sheet hole (441) and an adhesive portion (424). A separation member for separating the protective sheet (410) from the adhesive layer (115) may be attached to the adhesive portion (424). The first section (414) and the second section (416) can be sequentially separated from the adhesive layer (115) by a separation member, and can be separated from the adhesive layer (115) in a state of being connected to each other by the connection section (418). When the separation member is pulled while attached to the adhesive portion (424), the first section (414) is first separated from the adhesive layer (115), and then the connection section (418) and the second section (416) sequentially may be separated from the adhered layer (115).

The cut line (420) includes a main cut portion (421) extending from one side of the outer edge portion (412) to the connection section (418) to divide the side portion of the first section (414) and the side portion of the second section (416), and a sub-cut portion (422) connected to the end of the main cut portion (421) so as to be adjacent to the connection section (418). The sub-cut portion (422) may be arched, curved in another shape, or straight. In a case in which the sub-cut portion (422) is curved as shown, when the first section (414) is pulled in a direction in which the first section (414) is separated from the adhesive layer (115) by the separation member, the first section (414) can be separated from the connection section (418) more smoothly without being torn.

The shapes of each of the first section (414), second section (416), and connection section (418) of the protective sheet (410) according to this embodiment are not limited to those shown and may be changed in various ways. Additionally, the number or location of the adhesive portions (424) may be changed in various ways. Additionally, the formation location of the protective sheet hole (441) may be changed. Additionally, the main cut portion (421) may be curved.

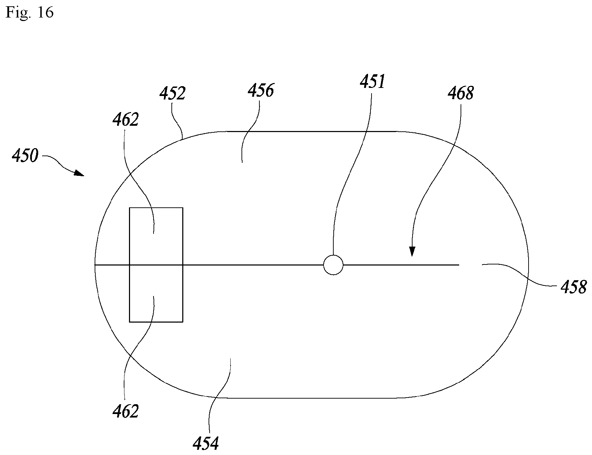

The protective sheet (450) shown in FIG. 16 includes a protective sheet hole (451) through which the insertion portion (113) of the sensor (112) can pass, a first section (454) and a second section (456) which are divided by a cut line (460) disposed inside the outer edge portion (452), and an adhesive portion (462) to which a separation member for separating the protective sheet (450) from the adhesive layer (115) is adhered. The first section (454) and the second section (456) are connected by a connection section (458). The first section (454) and the second section (456) are arranged so that their respective sides face each other. The protective sheet hole (451) is provided in such a way that a portion is placed in the first section (454) and the other portion is placed in the second section (456). The adhesive portion (462) is provided in the first section (454) and the second section (456), respectively. The adhesive portion (462) provided in the first section (454) and the adhesive portion (462) provided in the second section (456) are arranged to face each other with the cut line (460) interposed therebetween. The first section (454) and the second section (456) may be separated from the adhesive layer (115) by a separation member in a state of being connected by the connection section (458). When the separation member is pulled in a state of being attached to the adhesive portion (462), the first section (454) and the second section (456) may be separated from the adhesive layer (115) simultaneously or sequentially, and finally the connection section (458) may be separated from the adhesive layer (115).

The cut line (460) extends from one side of the outer edge portion (452) to the connection section (458) to divide the side of the first section (454) and the side of the second section (456). The cut line (460) may be formed in a straight line so as to pass through the protective sheet hole (451).

The protective sheet (450) according to this embodiment can be separated more quickly by the separation member by providing the adhesive portion (4620 in both the first section (454) and the second section (456).

The shapes of each of the first section (454), second section (456), and connection section (458) of the protective sheet (450) according to this embodiment are not limited to those shown and may vary in various ways. Additionally, the number or location of the adhesive portions (462) may be changed in various ways. Additionally, the formation location of the protective sheet hole (451) may be changed. Additionally, the cut line (460) may be curved.

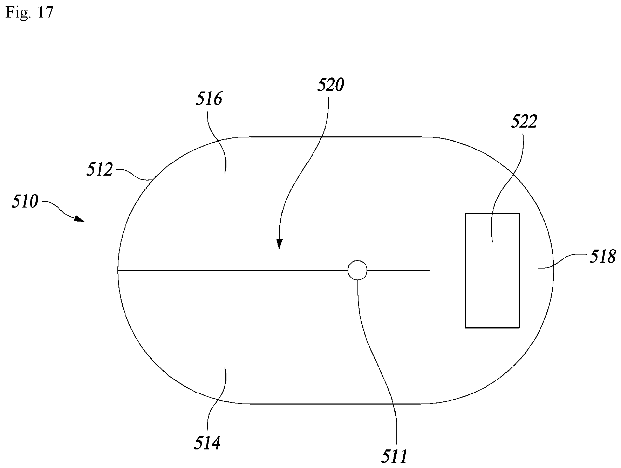

The protective sheet (510) shown in FIG. 17 includes a protective sheet hole (511) through which the insertion portion (113) of the sensor (112) can pass, a first section (514) and a second section (516) divided by a cut line (520) disposed inside the outer edge portion (512), and an adhesive portion (522) to which a separation member for separating the protective sheet (510) from the adhesive layer (115) is adhered. The first section (514) and the second section (516) are connected by a connection section (518). The first section (514) and the second section (516) are arranged so that their respective sides face each other. The protective sheet hole (511) is provided so that a portion is placed in the first section (514) and the other portion is placed in the second section (516). The adhesive portion (522) is provided on the connection section (518). The first section (514) and the second section (516) may be separated from the adhesive layer (115) by a separation member in a state of being connected by the connection section (518). When the separation member is pulled in a state of being attached to the adhesive portion (522), the connection section (518) may be separated from the adhesive layer (115) first, and then the first section (514) and the second section (516) may be separated simultaneously or sequentially.

The cut line (520) extends from one side of the outer edge portion (512) to the connection section (518) to divide the side of the first section (514) and the side of the second section (516). The cut line (520) may be formed in a straight line so as to pass through the protective sheet hole (511).

The protective sheet (510) according to the present embodiment can be more quickly separated by the separation member by providing the adhesive portion (522) at both the first section (514) and the second section (516).

The shapes of each of the first section (514), second section (516), and connection section (518) of the protective sheet (510) according to this embodiment are not limited to those shown and may be changed in various ways. Additionally, the number or location of the adhesive portions (522) may be changed in various ways. Additionally, the formation location of the protective sheet hole (511) may be changed. Additionally, the cut line (520) may be curved.

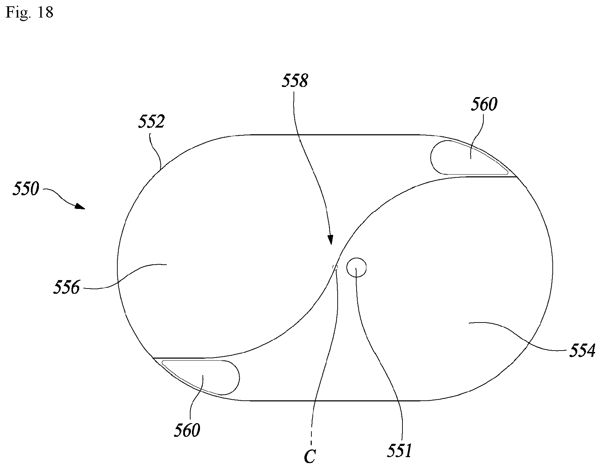

The protective sheet (550) shown in FIG. 18 includes a protective sheet hole (551) through which the insertion portion (113) of the sensor (112) can pass, a first section (554) and a second section (556) divided by a cut line (558) disposed inside the outer edge portion (552), and an adhesive portion (560) to which a separation member for separating the protective sheet (550) from the adhesive layer (115) is adhered. The first section (554) and the second section (556) are arranged so that their respective sides face each other. The protective sheet hole (551) is located in the first section (554).

The cut line (558) extends from one side of the outer edge portion (552) to the other side of the outer edge portion (552) so that the first section (554) and the second section (556) can be individually separated from the adhesive layer (115) without a part connected to each other. The cut line (558) is formed in a curved shape passing through the center (C) of the protective sheet (550). Here, the center (C) of the protective sheet (550) may be the center of gravity of the protective sheet (550). The cut line (558) is in a form that is point symmetrical with respect to the center (C) of the protective sheet (550). The first section (554) and the second section (556) divided by the cut line (558) are arranged to be point symmetrical with respect to the center (C) of the protective sheet (550).

Adhesive portions (560) are provided on both the first section (554) and the second section (556). A separation member for separating the protective sheet (550) from the adhesive layer (115) may be attached to the adhesive portion (560). When the separation member is pulled in a state of being attached to the adhesive portion (560), the first section (554) and the second section (556) may be individually separated from the adhesive layer (115). As shown, the adhesive portion (560) is provided at each end of the first section (554) and the second section (556), so that when the separation member is pulled, the first section (554) and the second section (556) can be more smoothly separated from the adhesive layer (115).

The shapes of each of the first section (554) and the second section (556) of the protective sheet (550) according to this embodiment are not limited to those shown and may be changed in various ways. Additionally, the number or location of the adhesive portions (560) may be changed in various ways. Additionally, the formation location of the protective sheet hole (551) may be changed. Additionally, the shape of the cut line (558) may be changed in various ways.

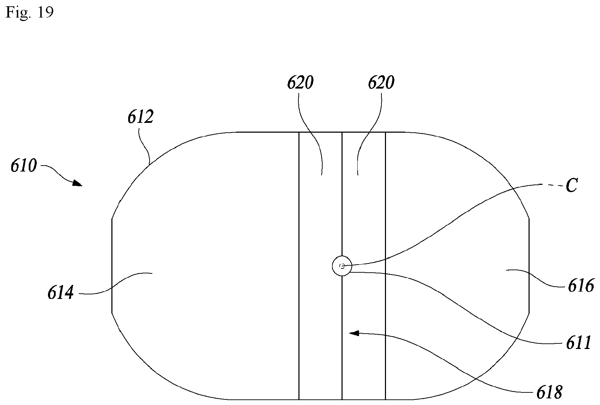

The protective sheet (610) shown in FIG. 19 includes a protective sheet hole (611) through which the insertion portion (113) of the sensor (112) can pass, a first section (614) and a second section (616) divided by a cut line (618) disposed inside the outer edge portion (612), and an adhesive portion (620) to which a separation member for separating the protective sheet (610) from the adhesive layer (115) is adhered. The first section (614) and the second section (616) are arranged so that their respective ends face each other. The protective sheet hole (611) is provided in such a way that a portion is located in the first section (614) and the other portion is located in the second section (616). The protective sheet hole (611) may be located at the center (C) of the protective sheet (610).

The cut line (618) extends from one side of the outer edge portion (612) to the other side of the outer edge portion (612) so that the first section (614) and the second section (616) can be individually separated from the adhesive layer (115) without a part connected to each other. The cut line (618) is formed in a straight line passing through the center (C) of the protective sheet (550). The first section (614) and the second section (616) may be symmetrically disposed on both sides of the cut line (618).

The adhesive portion (620) is provided on both the first section (614) and the second section (616). The adhesive portion (620) of the first section (614) and the adhesive portion (620) of the second section (616) are arranged to face each other with the cut line (618) interposed therebetween. A separation member for separating the protective sheet (610) from the adhesive layer (115) may be attached to the adhesive portion (620). When the separation member is pulled while attached to the adhesive portion (620), the first section (614) and the second section (616) may be individually separated from the adhesive layer (115).

The shapes of each of the first section (614) and the second section (616) of the protective sheet (610) according to this embodiment are not limited to those shown and may be changed in various ways. Additionally, the number or location of the adhesive portions (620) may be changed in various ways. Additionally, the formation location of the protective sheet hole (611) may be changed. Additionally, the shape of the cut line (618) may be changed to another shape, such as a straight shape.

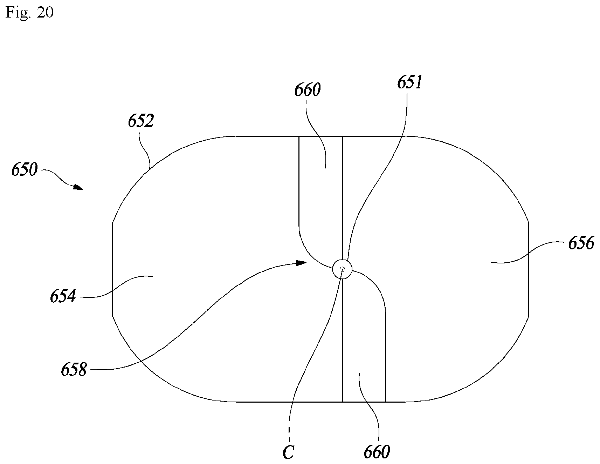

The protective sheet (650) shown in FIG. 20 includes a protective sheet hole (651) through which the insertion portion (113) of the sensor (112) can pass, a first section (654) and a second section (656) divided by a cut line (658) disposed inside the outer edge portion (652), and an adhesive portion (660) to which a separation member for separating the protective sheet (650) from the adhesive layer (115) is adhered. The first section (654) and the second section (656) are arranged so that their respective ends face each other. The protective sheet hole (651) is provided in such a way that a portion is located in the first section (654) and the other portion is located in the second section (656). The protective sheet hole (651) may be located at the center (C) of the protective sheet (650).

The cut line (658) extends from one side of the outer edge portion (652) to the other side of the outer edge portion (652) so that the first section (654) and the second section (656) can be individually separated from the adhesive layer (115) without a part connected to each other. The cut line (658) is formed in a curved shape passing through the center (C) of the protective sheet (650). The first section (654) and the second section (656) divided by the cut line (658) are arranged to be point symmetrical with respect to the center (C) of the protective sheet (650).

Adhesive portions (660) are provided on both the first section (654) and the second section (656). The adhesive portion (660) of the first section (654) and the adhesive portion (660) of the second section (656) are arranged to form point symmetry with respect to the center (C) of the protective sheet (650). A separation member for separating the protective sheet (650) from the adhesive layer (115) may be attached to the adhesive portion (660). When the separation member is pulled in a state of being attached to the adhesive portion (660), the first section (654) and the second section (656) may be individually separated from the adhesive layer (115).

The shapes of each of the first section (654) and the second section (656) of the protective sheet (650) according to this embodiment are not limited to those shown and may be changed in various ways. Additionally, the number or location of the adhesive portions (660) may be changed in various ways. Additionally, the formation location of the protective sheet hole (651) may be changed. Additionally, the cut line (658) may be changed to another shape, such as a straight line.

Although the present disclosure has been described above with preferred examples, the scope of the present disclosure is not limited to the form described and shown above.

For example, the drawing shows that the protective sheet includes a first section and a second section divided by a cut line, but the protective sheet may be divided into three sections or more than three sections.

In addition, in the previous example, a body attachable device was described as measuring the blood glucose of a user by being attached to the skin of a user, but the body attachable device according to the present disclosure may be configured to measure various biometric information of the user by being attached to the skin of a user. Additionally, the body attachable device according to the present disclosure may have various other configurations that can be used by being attached to the skin of a user, in addition to the purpose of measuring the biometric

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

## Claims

1. A protective sheet for a body attachable device detachably coupled to an adhesive layer to cover and protect the adhesive layer of the body attachable device configured to be attachable to skin of a user, the protective sheet for the body attachable device comprising:
a first section and a second section divided by a cut line disposed inside an outer edge portion of the protective sheet; and
an adhesive portion provided at at least one of the first section and the second section such that a separation member configured to be pullable in a direction of separating the protective sheet from the adhesive layer is adhered.

2. The protective sheet for the body attachable device according to claim 1,
comprising a connection section connecting the first section and the second section,
wherein the first section and the second section are configured to be separatable from the adhesive layer in a state of being connected to each other by the connection section while being sequentially separatable from the adhesive layer by the separation member.

3. The protective sheet for the body attachable device according to claim 2,
wherein the connection section comprises
a middle section disposed inside the second section and surrounding the first section;
a first section connection portion connecting the first section and the middle section; and
a middle connection portion connecting the middle section and the second section, and wherein the cut line comprises
a first section cut line dividing the first section and the middle section; and
a middle section cut line dividing the second section and the middle section.

4. The protective sheet for the body attachable device according to claim 3,
wherein the first section cut line comprises
a first section end cut portion dividing one end of the first section and the middle section; and
a pair of first section side cut portions extending from both ends of the first section end cut portion toward the outer edge portion, respectively, and
wherein the middle section cut line comprises
a middle end cut portion dividing one end of the middle section and the second section; and
a pair of middle side cut portions extending from both end portions of the middle end cut portion toward the outer edge portion, respectively.

5. The protective sheet for the body attachable device according to claim 4,
wherein the first section cut line comprises
a pair of first section connection end cut portions connected to the pair of first section side cut portions, respectively, to be disposed to be symmetrical to each other with respect to the first section connection portion,
wherein the middle section cut line comprises
a pair of middle section connection end cut portions connected to the pair of middle side cut portions, respectively, to be disposed to be symmetrical to each other with respect to the middle connection portion, and
wherein the pair of first section connection end cut portions and the pair of middle section connection end cut portions are formed in a curved shape.

6. The protective sheet for the body attachable device according to claim 2,
wherein the second section is arranged to surround the first section, and
wherein the cut line comprises
a first section end cut portion dividing one end of the first section and the second section; and
a pair of first section side cut portions extending from both ends of the first section end cut portion toward the outer edge portion, respectively.

7. The protective sheet for the body attachable device according to claim 6,
wherein the cut line comprises
a cross cut portion extending from the outer edge portion toward the first section and connected to one of the pair of first section side cut portions.

8. The protective sheet for the body attachable device according to claim 2,
wherein the second section is arranged to surround the first section, and
wherein the cut line comprises
a first cut portion having a curved shape surrounding the first section, and
a second cut portion connected to the first cut portion and extending to the outer edge.

9. The protective sheet for the body attachable device according to claim 2,
wherein the first section and the second section are arranged such that respective ends of the first section and the second section face each other, and the connection section is arranged to face a side of the first section and a side of the second section, and
wherein the cut line comprises
a main cut portion dividing the connection section into the side portion of the first section and the side portion of the second section; and
a cross cut portion connected to the main cut portion by extending from the outer edge portion toward the main cut portion to divide the end of the first section and the end of the second section.

10. The protective sheet for the body attachable device according to claim 2,
wherein a side of the first section and a side the second section are arranged to face each other, and
wherein the cut line extends from one side of the outer edge portion to the connection section to divide the side of the first section and the side of the second section.

11. The protective sheet for the body attachable device according to claim 1,
wherein the cut line extends from one side of the outer edge portion to an other side of the outer edge portion such that the first section and the second section are individually separatable from the adhesive layer without any part connected to each other, and
wherein the adhesive portion is provided in the first section and the second section, respectively.

12. The protective sheet for the body attachable device according to claim 11,
wherein the first section and the second section are arranged to be point-symmetrical with respect to a center of the protective sheet.

13. The protective sheet for the body attachable device according to claim 1, comprising:
a protective sheet hole formed inside the outer edge portion to penetrate the protective sheet in a thickness direction so that a sensor, included in the body attachable device to be insertable into the skin of the user, passes therethrough.

14. A body attachable device configured to be attachable to skin of a user, the body attachable device comprising:
a housing;
an adhesive layer provided at one side of the housing to be attachable to the skin of the user; and
a protective sheet detachably coupled to the adhesive layer to cover and protect the adhesive layer,
wherein the protective sheet comprises
a first section and a second section divided by a cut line disposed inside an outer edge portion of the protective sheet; and
an adhesive portion provided at at least one of the first section and the second section such that a separation member configured to be pullable in a direction of separating the protective sheet from the adhesive layer is adhered.
